# EUROPÄISCHE PATENTANMELDUNG

(11) **EP 3 403 596 A1**
(43) Veröffentlichungstag der Anmeldung: **21.11.2018**
(21) Anmeldenummer: 17171245.8
(22) Anmeldetag: 16.05.2017
(51) Int. Cl.: A61B 17/12

(54) **IMPLANTATIONS- UND VERANKERUNGSSYSTEM FÜR EINEN VORHOFOHROKKLUDER**

(71) Anmelder: Universitätsklinikum Jena, 07743 Jena (DE)
(72) Erfinder: PÖRNER, Tudor Constantin, 68165 Mannheim (DE)
(74) Vertreter: Isfort, Olaf

(57) **Zusammenfassung**

Die Erfindung betrifft ein Implantat zum Verschließen hohler Strukturen im menschlichen oder tierischen Körper mittels endovaskulärer Intervention, mit einem Okkluder mit einer aus einer Vielzahl von Streben gebildeten, im expandierten Zustand im Wesentlichen korb- oder scheibenförmigen und im komprimierten Zustand im Wesentlichen rohrförmigen Struktur, einem Verankerungssystem mit einem Beabstandungssystem und einem Tentakelsystem, wobei das Verankerungssystem lösbar mit dem Okkluder verbunden ist, und einem Einführelement, das über ein Ablöseelement trennbar mit dem proximalen Teil des Okkluders verbunden ist, wobei das Beabstandungssystem im Wesentlichen ein rohr- und/oder stabförmig ausgestaltetes Distanzelement mit einem ersten und einem zweiten Ende umfasst, wobei das Distanzelement an seinem ersten Ende ein erstes Festlegungsmittel umfasst mit dem das Verankerungssystem kraft- und/oder formschlüssig mit dem Okkluder verbindbar ist und an seinem zweiten Ende das Tentakelsystem umfasst und der Okkluder ein zweites Festlegungsmittel umfasst mit dem das Verankerungssystem kraft- und/oder formschlüssig mit dem Okkluder verbindbar ist.

## Beschreibung

Die Erfindung betrifft ein Implantat für Herzohren, insbesondere ein Implantat mit einem variablen Verankerungssystem.

Die Herz- oder auch Vorhofohren (*auriculae atrii*) sind kleine Ausstülpungen der Herzvorhöfe. Sie gehören zur normalen Anatomie des menschlichen Herzens und sind *per se* nicht pathologisch. Jeweils ein Herzohr befindet sich im rechten und eines im linken Vorhof. Beim gesunden Menschen kontrahieren die Herzohren im normalen Rhythmus des Herzens, sie werden zudem vom Blutstrom der Vorhöfe durchspült und tauschen so ihr Blutvolumen regelmäßig aus.

Zu pathologischen Zuständen kann es kommen, wenn die Herzohren nicht oder nur unvollständig durchspült werden, sie also nicht in gewohnter Art und Weise kontrahieren und/oder die Vorhöfe keinen ausreichenden Blutstrom schaffen. Dies kann bis hin zu einer Stagnation des Blutflusses, einer Hämostase, im Herzohr und in der Folge zur Bildung von Thromben im Herzohr führen.

Die Ursachen hierfür sind vielfältig, jedoch ist der beschriebene Zustand häufig die Folge eines Vorhofflimmerns, also einer speziellen Art der Herzrhythmusstörung. Besonders betroffen ist das linke Herzohr (*auricula sinistra*), das neben dem Stamm der Lungenarterie liegt. Thromben, die sich hier bilden und dann als Emboli aus dem Herzen gespült werden, können zu Schlaganfällen oder sonstigen Verschlüssen im Gefäßsystem führen.

Um diesen möglichen Folgen eines Vorhofflimmerns vorzubeugen, erhalten betroffene Patienten, neben der ursächlichen Behandlung der Rhythmusstörung, in der Regel eine Embolieprophylaxe in Form einer medikamentösen Antikoagulation. Diese geht jedoch immer mit einem erhöhten Blutungsrisiko einher, sodass immer eine individuelle Risikoanalyse erfolgen muss.

Alternative Behandlungsmethoden stehen inzwischen vor allem in Form sogenannter LAA-Okkluder (**l**eft **a**trial **a**ppendage = linkes Herzohr) zur Verfügung. Diese scheiben- oder schirmchenartigen Implantate können endovaskulär durch einen Katheter in das Herzohr vorgeschoben und dort aufgespannt werden. Der Schirm oder die Scheibe, deren Gerüst in der Regel aus einem Formgedächtnismaterial besteht und mit einer Membran überspannt ist, dichtet das Herzohr zum Vorhof ab. Das Implantat wird in kurzer Zeit von Endothelzellen überwachsen.

Neben der zuverlässigen Abdichtung des Herzohrs gegenüber dem Vorhof ist vor allem der feste Sitz des Implantats im Herzohr entscheidend für den interventionellen Erfolg. Löst sich das Implantat nämlich in der Folge aus dem Herzohr, kommt es mit hoher Wahrscheinlichkeit zu lebensbedrohlichen Komplikationen.

Im Stand der Technik sind unterschiedliche LAA-Okkluder bekannt, die durch ihre spezielle Form und/oder durch zusätzliche Verankerungselemente einen sicheren Sitz im Herzohr garantieren sollen.

Die EP 2 074 953 B1 offenbart einen im wesentlichen zweigeteilten LAA-Okkluder, wobei der proximale Teil scheibenförmig ist, am Eingang des Herzohrs zum Vorhof hin aufliegt und das Herzohr so gegenüber dem Vorhof abdichtet. Der distale Teil ist zylindrisch geformt und verankert das Implantat im Herzohr. Randständig im distalen Teil befinden sich zusätzliche eingebrachte Verankerungselemente, die nach proximal umgebogen sind und so einer eventuellen Bewegung des Implantats Richtung Vorhof entgegenwirken. Ein ähnliches Implantat bestehend aus zwei Schirmen offenbart die EP 1 948 030 B1, wobei keine speziellen Verankerungselemente beschrieben werden.

Die WO 2015/079023 A1 offenbart ein aus Streben geformtes im Wesentlichen korbförmiges Implantat, dessen geschlossenes Ende die Verbindung zwischen Herzohr und Vorhof verschließt und an dessen offenem Ende sich Elemente zur Verankerung des Implantats im Herzohr befinden.

Die EP 1 135 068 B1, EP 1 441 649 B1 und EP 1 605 865 B1 offenbaren LAA-Okkluder mit verschiedenen Implantations- und Festlegungshilfen. Im Wesentlichen soll durch die Hilfsmittel dem Operateur die Möglichkeit gegeben werden, den Okkluder reversibel von einem expandierten in einen geschlossenen Zustand zu überführen, um so eine bestmögliche Platzierung durch ein mögliches Repositionieren zu erreichen. Dies wird im Wesentlichen durch zumindest ein zentrales axiales Element erreicht, mit dem der Abstand zwischen proximalem und distalem Ende des Implantates und somit auch dessen Durchmesser verändert werden kann. Als Verankerungselemente offenbaren diese Dokumente beispielsweise Widerhaken an den Streben des Implantates oder die Festlegung des eher scheibenförmigen proximalen Abschnitts durch einen Stent-ähnlichen distalen Abschnitt, ähnlich wie in der EP 2 074 953 B1 beschrieben.

Nachteilig an den bekannten Implantaten ist, dass diese nur bedingt an die Anatomie des Herzohrs anpassungsfähig sind, nämlich allein durch die Auswahl des passenden Schirmdurchmessers. Somit kann bei der Auswahl eines bekannten Implantats nur ein Teil der Anatomie des Herzohres berücksichtigt werden, nämlich der Durchmesser der Öffnung. Die individuelle Tiefe des Herzohrs, die von weniger als 2 cm bis über 4 cm betragen kann, bleibt unberücksichtigt. Doch gerade die Anpassung eines Implantats mit einem zusätzlichen, distalen Verankerungssystem an die Tiefe des Herzohres ist ein weiterer wesentlicher Faktor für eine zuverlässige Verankerung des Implantats. Ein optimaler Sitz kann mit vorbekannten Implantaten ohne variable Längeneinstellung nicht erreicht werden, was die Gefahren entweder einer Verletzung des Herzohres oder einer Dislokation, also des Ver- oder Herausrutschens, des Implantats und in der Folge möglicher Embolien erhöhen.

Es ist daher eine Aufgabe der Erfindung, ein Implantat zur Verfügung zu stellen, das die oben genannten Nachteile überwindet. Hierfür soll das Implantat insbesondere an die Anatomie des zu okkludierenden Herzohrs anpassbar sein.

Gelöst wird diese Aufgabe durch ein Implantat mit den Merkmalen des Anspruchs 1. Vorteilhafte Ausgestaltungen sind jeweils Gegenstand der abhängigen Ansprüche. Es ist darauf hinzuweisen, dass die in den Ansprüchen einzeln aufgeführten Merkmale auch in beliebiger und technologisch sinnvoller Weise miteinander kombiniert werden können und somit weitere Ausgestaltungen der Erfindung aufzeigen.

Bei dem erfindungsgemäßen Implantat handelt es sich um ein Implantat zum Verschließen hohler Strukturen im menschlichen oder tierischen Körper, welches kathetergestützt endovaskulär eingebracht wird. Das Implantat umfasst einen Okkluder mit einer aus einer Vielzahl von Streben gebildeten, im expandierten Zustand im Wesentlichen scheiben- oder korbförmigen und im komprimierten Zustand im Wesentlichen rohrförmigen Struktur, ein Verankerungssystem - umfassend ein Beabstandungssystem und ein Tentakelsystem - und ein Einführelement, das über ein Ablöseelement trennbar mit dem proximalen Teil des Okkluders verbunden ist.

Bei einem erfindungsgemäßen Implantat ist die Gesamtlänge des Implantats durch ein längenvariables Verankerungssystem veränderbar. Die Längenvariabilität wird im Wesentlichen durch zwei Systeme realisiert:
Bei dem bevorzugten System ist das Verankerungssystem insgesamt austauschbar und es stehen erfindungsgemäß alternative Verankerungssysteme mit Beabstandungssystemen unterschiedlicher Länge zur Verfügung.

Bei einem weiteren System umfasst das Verankerungssystem ein längenverstellbares Beabstandungssystem mit mindestens zwei Distanzelementen, die innerhalb eines Stellbereichs, in dem sie sich zumindest teilweise überlappen, zur Längenanpassung teleskopartig ineinandergeschoben werden können.

Die nachfolgenden Beschreibungen bevorzugter Ausführungsformen erfindungsgemäßer Implantate beziehen sich, sofern nicht ausdrücklich Anderes erwähnt wird, auf den frei expandierten Zustand des Implantats an der Luft und nicht auf den nach einer Implantation gegebenenfalls erreichbaren expandierten Zustand.

Die Lage- und Richtungsbezeichnungen *proximal* und *distal* sind von der ursprünglichen Lage des Implantats im Katheter abzuleiten. So bezeichnet *proximal* die dem Operateur zugewandte Seite des Implantats beziehungsweise die zum Operateur weisende Richtung und *distal* die vom Operateur abgewandte Seite des Implantats beziehungsweise die vom Operateur wegweisende Richtung.

In einer bevorzugten Ausführungsform des erfindungsgemäßen Implantats ist die Gesamtlänge des Implantats durch ein längenvariables Verankerungssystem veränderbar. Die Gesamtlänge ist bei der bevorzugten Ausführungsform durch ein insgesamt austauschbares Verankerungssystem einstellbar. Das Verankerungssystem umfasst das Beabstandungssystem und das Tentakelsystem. Es stehen unterschiedlich lange Verankerungssysteme zur Verfügung, wobei die unterschiedliche Länge der Verankerungssysteme durch die unterschiedliche Länge der jeweiligen Beabstandungssysteme erreicht wird. Bei dieser bevorzugten Ausführungsform des erfindungsgemäßen Implantats umfasst das Beabstandungssystem ein Distanzelement.

Somit kann durch die Erfindung ein Implantatsystem bereitgestellt werden, mit einem Satz aus zwei oder mehr Verankerungssystemen, die sich hinsichtlich der Länge voneinander unterscheiden. Entsprechend der Anatomie des Patienten kann aus dem Satz das passende Verankerungssystem ausgewählt werden.

Das Distanzelement ist im Wesentlichen stab- und/oder rohrförmig ausgebildet mit einem ersten und einem zweiten Ende. Am ersten Ende umfasst das Distanzelement ein erstes Festlegungsmittel zur Festlegung des Verankerungssystems am Okkluder.

Das Distanzelement ist im Wesentlichen aus den gleichen Materialien gefertigt wie der Okkluder. Bevorzugt sind Nitinol-Verbindungen, Federstahle oder sonstige Formgedächtnismaterialien, beispielsweise auch entsprechende Polymere. Das Distanzelement kann beispielsweise aus einem Rohr geschnitten oder auch aus Filamenten geflochten sein.

Um das Implantat insgesamt im Gefäßsystem möglichst gut manövrierbar zu halten, sollte das distale Ende des Beabstandungssystems im komprimierten Zustand maximal bis zum distalen Ende der Mehrzahl der Streben des Okkluders reichen. Insofern wird möglichst das komplette Verankerungssystem inklusive der Verankerungselemente im komprimierten Zustand vom Okkluder überdeckt.

Der Okkluder umfasst ein dem ersten Festlegungsmittel des Distanzelements entsprechendes zweites Festlegungsmittel zur Festlegung des Verankerungssystems über das Distanzelement. Dieses zweite Festlegungsmittel ist bevorzugt einstückig mit zumindest Teilen des Okkluders gefertigt. Es sind jedoch auch Ausführungsformen möglich, bei denen das zweite Festlegungsmittel getrennt angefertigt und anschließend mit dem Okkluder fest verbunden wird. Entsprechende Verbindungstechniken wir Kleben, Schweißen, Pressen etc. sind dem Fachmann hinlänglich bekannt.

Als Festlegungsmittel sind beispielsweise form- und/oder kraftschlüssige Verbindungen denkbar, die zu einer lösbaren Verbindung zwischen Okkluder und Ankersystem führen. Prinzipiell denkbar sind auch nicht lösbare Verbindungen, jedoch sind lösbare Verbindungen zu bevorzugen. Vorteilhaft bei lösbaren Verbindungen ist, dass ein einmal eingesetztes unpassendes oder fehlerhaftes Ankersystem nicht destruktiv austauschbar ist. Insbesondere ist eine Schraubverbindung bevorzugt, bei der der Okkluder an zentraler Stelle, in der Regel dort, wo die Streben des Schirms zusammenlaufen, an der distalen Seite ein Innengewinde aufweist und das Beabstandungssystem am proximalen Ende des an den Okkluder anschließenden Distanzelements ein entsprechendes Außengewinde aufweist. Andere Festlegungsmittel wie beispielsweise Rasten, Schnapp- oder Klickverschlüsse sind ebenfalls denkbar. Dem Fachmann sind hier verschiedenste Möglichkeiten bekannt.

An seinem zweiten Ende umfasst das Distanzelement ein Tentakelsystem, das nachfolgend noch detailliert beschrieben wird.

Zur besseren Verankerung im Herzohr umfasst das Verankerungssystem ein Tentakelsystem, welches sich bevorzugt am distalen Ende des Verankerungssystems befindet. Ein solches Tentakelsystem ist insbesondere für ein erfindungsgemäßes Implantat geeignet, jedoch kann das Tentakelsystem auch an anderen Implantaten, insbesondere an anderen LAA-Okkludern, angebracht werden und deren Sitz verbessern. Insofern ist die Anbringung eines solchen Tentakelsystems auch nicht auf solche Implantate mit einer erfindungsgemäßen Längenverstellung beschränkt.

Das erfindungsgemäße Tentakelsystem umfasst zwei bis sechzehn, bevorzugt sechs bis zwölf und insbesondere acht Verankerungselemente. Das Tentakelsystem kann aus einem Stück gefertigt sein, es ist jedoch auch denkbar, dass das Tentakelsystem aus einzelnen Teilen, beispielsweise aus einzelnen Verankerungselementen, gefertigt und dann zusammengesetzt wird.

Die Verankerungselemente sind am Implantat bevorzugt im gleichen Abstand auf einer Ebene um das Distanzelement angeordnet. Es sind jedoch auch Ausführungsformen denkbar, bei denen die Verankerungselemente in mehreren Ebenen und/oder unterschiedlich voneinander beabstandet angelegt sind.

Das Tentakelsystem und das Beabstandungssystem können aus einem Stück gefertigt, insbesondere aus einem Rohr geschnitten sein. Umfasst das Beabstandungssystem mehrere Distanzelemente, dann ist das Tentakelsystem bevorzugt an dem am weitesten distal befindlichen Distanzelement festgelegt oder mit diesem aus einem Stück gefertigt, insbesondere aus einem Rohr geschnitten. Das Beabstandungssystem und das Tentakelsystem können auch aus mehreren Teilen verbindbar gefertigt sein. Dabei können das Beabstandungssystem und das Tentakelsystem direkt oder über mindestens ein weiteres Teil, beispielsweise ein Ringelement, miteinander verbindbar sein. Entsprechende Mittel zur Festlegung der einzelnen Teile sind dem Fachmann bekannt.

Die Verankerungselemente umfassen einen proximalen Hauptbereich, einen distalen Endbereich und einen zwischen Haupt- und Endbereich liegenden Nebenbereich.

Die Länge des Hauptbereichs beträgt bevorzugt zwischen 10 und 20 mm, insbesondere 15 mm und die Länge des Nebenbereichs bevorzugt zwischen 1 und 10 mm, insbesondere 5 mm. Um eine Perforation des relativ dünnen Herzohrgewebes durch den distalen Bereich des Verankerungselements zu vermeiden, ist der Endbereich atraumatisch, insbesondere in Form einer Schlaufe, geformt, wobei der Durchmesser der Schlaufe zwischen 1 und 5 mm, insbesondere 3 mm beträgt. Um die Verankerung weiter zu verbessern, können in einer weiterhin bevorzugten Ausführungsform alle oder einzelne dieser Schlaufen ein oder mehrere Festlegungselemente umfassen. Die Länge des Festlegungselements beträgt zwischen 0,5 und 5 mm, bevorzugt zwischen 1 und 3 mm und insbesondere 2 mm. In einer bevorzugten Ausführungsform umfassen alle Schlaufen jeweils ein Festlegungselement, in einer weiterhin bevorzugten Ausführungsform weisen die Festlegungselemente die Form eines Widerhakens auf.

Der proximale Hauptbereich bildet im frei expandierten Zustand mit der durch die Distanzelemente gebildeten Längsachse einen nach außen gerichteten Winkel zwischen 90 und 150 Grad, bevorzugt von 105 Grad und der Nebenbereich bildet mit dem Hauptbereich im frei expandierten Zustand einen nach außen gerichteten Winkel zwischen 90 und 150 Grad, bevorzugt von 105 Grad.

Das erfindungsgemäße Implantat umfasst eine im Wesentlichen einem endovaskulären Katheter entsprechende Einführeinheit (*Occluder Delivery System, ODS*), in der sich das Implantat im komprimierten Zustand befindet. In dem ODS kann das Implantat durch einen weiteren Katheter zum Behandlungsort vorgeschoben werden. Um das ODS bis an den Behandlungsort bringen zu können, ist das ODS länger als der weitere Katheter. Am distalen Ende umfasst das ODS mindestens einen röntgendichten Marker. Im distalen Abschnitt proximal der Marker umfasst das ODS mindestens eine seitliche Perforation, die das innere Lumen des ODS' mit dem äußeren Lumen verbindet. Diese Anordnung hat den Hauptvorteil, dass mittels Kochsalzlösungapplikation eine zusätzliche echokardiografische Lagekontrolle vor Platzierung durchgeführt werden kann. Somit wird die sonst übliche Gabe von Kontrastmittel erspart und die Implantationssicherheit erhöht. Weitere Vorteile bestehen darin, dass eventuell noch in dem ODS Lumen befindliche Luft besser aus dem ODS gespült werden kann.

Das Implantat umfasst weiterhin ein im Wesentlichen langgestrecktes Einführelement (*Release Bar, RB*) mit einem am distalen Ende des Einführelements angeordneten Ablöseelement (*Release Mechanism, RM).* Um das Implantat implantieren zu können, ist das Einführelement länger als das ODS. Das Ablöseelement ist über einen Ablösemechanismus lösbar mit dem proximalen Teil des Okkluders verbunden. Entsprechende Ablösemechanismen sind dem Fachmann bekannt, wie beispielsweise thermische, chemische, elektrische, elektrolytische oder mechanische Ablösemechanismen.

In einer bevorzugten Ausführungsform umfasst das Implantat weiterhin ein Sicherungssystem mit mindestens einem Sicherungselement (*Rescue Leash, RL*) und mindestens einem Befestigungselement für das Sicherungselement. Das Sicherungssystem eignet sich insbesondere für ein erfindungsgemäßes Implantat, es ist jedoch denkbar, ein solches Sicherungssystem auch an anderen Implantaten anzubringen, insbesondere anderen endovaskulären Implantaten. Entsprechend ist die Verwendung eines solchen Sicherungssystems nicht auf einen erfindungsgemäßen LAA-Okkluder und insbesondere auch nicht auf LAA-Okkluder im Allgemeinen beschränkt.

Das Befestigungselement ist bevorzugt am proximalen Ende des Implantats festlegbar. Das Sicherungselement ist lösbar mit dem Befestigungselement verbindbar. Das Sicherungselement dient dazu, das Implantat auch nach kompletter Ablösung des Ablöseelements noch in den Katheter, in dem das Implantat zum Behandlungsort vorgebracht wurde, zurückziehbar zu halten.

In einer bevorzugten Ausführungsform umfasst das Befestigungselement ein Loch oder eine Öse, durch die das Sicherungselement durchführbar ist. Das Sicherungselement umfasst ein Filament. Das Filament kann aus einem für endovaskuläre Methoden geeigneten Material gefertigt sein, wie beispielsweise aus einem Metall, einer geeigneten Legierung oder aus einer nicht resorbierbaren Polymerverbindung. Dem Fachmann sind weitere geeignete Materialien bekannt.

Das Sicherungselement ist bevorzugt zumindest einmal so um oder durch das Befestigungselement geführt, dass das Sicherungselement durch Zug an einem seiner Enden, aber nicht durch Zug an beiden Enden vom Befestigungselement gelöst werden kann. Das Sicherungselement ist so lang, dass seine beiden Enden gut erreichbar für den Operateur aus dem proximalen Ende des eingesetzten Katheters reichen.

Das Sicherungselement verläuft in einer bevorzugten Ausführungsform innerhalb des Einführelements, die in diesem Fall schlauchförmig gestaltet ist. Die Enden des Sicherungselements ragen entsprechend aus dem proximalen Ende des Einführelements heraus, insbesondere auch noch nach der Ablösung des Ablöseelements. Es sind auch Ausführungsformen denkbar, bei denen das Sicherungselement zumindest teilweise außerhalb des Einführelements verläuft.

Das erfindungsgemäße Implantat weist gegenüber dem Stand der Technik eine Vielzahl von Vorteilen auf. So kann das Implantat durch die Längeneinstellung wesentlich besser an die individuelle Anatomie des zu verschließenden Herzohrs angepasst werden, was zu einem deutlich verbesserten Sitz führt. Die Gefahr, dass das Implantat verrutscht oder sich gar aus dem Herzohr löst und zu lebensbedrohlichen Zuständen, insbesondere Embolien, führt, ist minimiert.

Das optimierte Verankerungssystem mit den mehrfach gewinkelten Verankerungselementen sorgt zudem für einen verbesserten Sitz des Implantats.

Das Sicherungselement erhöht die Produktsicherheit während der Intervention zusätzlich. Stellt sich nämlich bei vorbekannten Implantaten nach vollständiger Ablösung des Einführelements heraus, dass der Sitz des Implantats nicht optimal ist oder sich das Implantat gar aus dem Herzohr zu lösen droht, kann das Implantat nicht schnell endovaskulär geborgen werden, was schlimmstenfalls einen offenchirurgischen Eingriff am Herzen zur Entfernung des Implantats notwendig macht. Entgegen kann das Implantat mit dem erfindungsgemäßen Sicherungselement unverzüglich in den verwendeten und noch liegenden Katheter zurückgezogen werden, wodurch weitere Komplikationen nahezu ausgeschlossen sind.

Die Erfindung sowie das technische Umfeld werden nachfolgend anhand der Figuren näher erläutert. Es ist darauf hinzuweisen, dass die Figuren eine besonders bevorzugte Ausführungsvariante der Erfindung zeigen. Die Erfindung ist jedoch nicht auf die gezeigte Ausführungsvariante beschränkt. Insbesondere umfasst die Erfindung, soweit es technisch sinnvoll ist, beliebige Kombinationen der technischen Merkmale, die in den Ansprüchen aufgeführt oder in der Beschreibung als erfindungsrelevant beschrieben sind.

Es zeigen:
- Fig.1: einen Längsschnitt durch eine erste Ausführungsform des erfindungsgemäßen Implantats mit Verankerungssystem im frei expandierten Zustand.
- Fig. 2: einen Längsschnitt durch eine erste Ausführungsform des erfindungsgemäßen Implantats mit Verankerungssystem im komprimierten Zustand mit Zuführkatheter, Katheter, Zuführelement und Sicherungselement.
- Fig. 3: einen Längsschnitt gemäß Figur 2, wobei das Implantat komplett aus dem Zuführkatheter vorgeschoben ist.
- Fig. 4: einen Längsschnitt gemäß Figur 3, wobei das Implantat vom Zuführelement gelöst, ist, jedoch vom Sicherungselement gehalten wird.
- Fig. 5: einen Längsschnitt gemäß Figur 4, wobei das Implantats nun auch vom Sicherungselement gelöst ist.

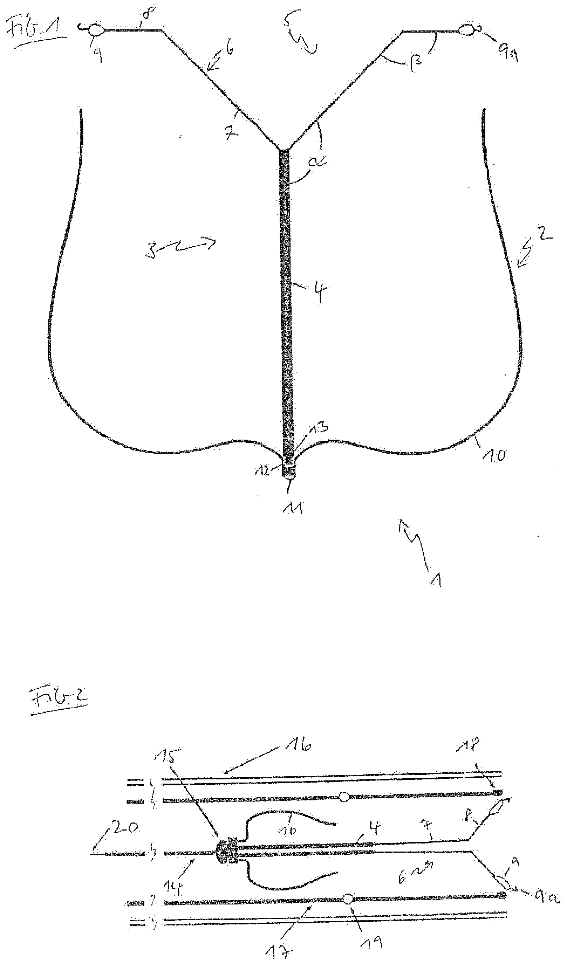

Figur 1 zeigt einen Längsschnitt, insbesondere die durch einen zentral geführten Längsschnitt durch eine erste Ausführungsform des erfindungsgemäßen Implantats 1 erzeugte Schnittebene. Das Implantat 1 ist im Wesentlichen in seinem ungezwungen expandierten Zustand an der Luft dargestellt.

Das Implantat 1 umfasst im Wesentlichen einen Okkluder 2 und ein Verankerungssystem 3. Der Okkluder 2 ist im Wesentlichen aus Streben 10 schirmförmig aufgebaut. Die Streben 10 laufen zumindest überwiegend in einem zentralen Bereich zusammen. Der Okkluder 2 kann bevorzugt aus einem Rohr geschnitten sein, wobei auch Ausführungsformen denkbar sind, bei denen der Okkluder 2 im Wesentlichen geflochten ist. Der zentrale Bereich kann beispielsweise ein nicht geschnittener Bereich des ursprünglichen Rohrs sein. Es kann sich hierbei allerdings auch um eine eigene Struktur handeln, die mit dem Fachmann bekannten Mitteln an dem schirmförmigen Bereich des Okkluders 2 festlegbar ist. Vorzugsweise an diesem zentralen Bereich befindet sich auf der distalen Seite das Festlegungsmittel 12 zur lösbaren Befestigung des Verankerungssystems 3. In einer bevorzugten Ausführungsform ist das Festlegungsmittel 12 des Okkluders 2 als Innengewinde ausgestaltet. Auf der proximalen Seite im zentralen Bereich befindet ein Befestigungselement 11 zur lösbaren Anbringung des Sicherungselements 20 (in den folgenden Figuren dargestellt). Zudem befindet sich an der proximalen Seite im zentralen Bereich ein Verbindungselement zur trennbaren Festlegung des Ablöseelements 15 (in den folgenden Figuren dargestellt).

Das Verankerungssystem 3 umfasst ein Beabstandungssystem 4 und ein Tentakelsystem 5. Das Beabstandungssystem 4 umfasst in der dargestellten Ausführungsform ein Distanzelement. Es sind jedoch auch Ausführungsformen denkbar, bei denen das Beabstandungssystem 4 mehrere Distanzelemente umfasst, die gegebenenfalls über einen Beabstandungsmechanismus gegeneinander verstellbar sind und so eine Längeneinstellung des Beabstandungssystems 4 ermöglichen.

In der dargestellten Ausführungsform umfasst das Distanzelement 4 an seinem proximalen Ende ein Festlegungsmittel 13 zur lösbaren Befestigung des Verankerungssystems 3 am Festlegungsmittel 12 des Okkluders 2. Bevorzugt ist das Festlegungsmittel 13 ein Außengewinde und das Festlegungsmittel 12 ein passendes Innengewinde. Es sind jedoch auch andere lösbare Befestigungen denkbar. Am distalen Ende des Distanzelements 4 ist das Tentakelsystem 5 festlegbar. Distanzelement 4 und Tentakelsystem 5 können in einer bevorzugten Ausführungsform aus einem Stück gefertigt, beispielsweise aus einem Rohr geschnitten sein. Es sind jedoch auch Ausführungsformen denkbar, bei denen Distanzelement 4 und Tentakelsystem 5 aus verschiedenen Stücken gefertigt sind und anschließend mit bekannten Methoden verbunden werden.

Das Tentakelsystem 5 umfasst mindestens zwei Verankerungselemente 6. Die Verankerungselemente 6 gliedern sich vorzugsweise in drei Bereiche, einen Hauptbereich 7, einen Nebenbereich 8 und einen Endbereich 9. Der Winkel α zwischen Beabstandungssystem 4 und Hauptbereich 7 beträgt bevorzugt 105°, andere Winkel sind denkbar. Der Winkel β zwischen Hauptbereich 7 und Nebenbereich 8 beträgt bevorzugt 105°, andere Winkel sind denkbar. Der Endbereich 9 ist bevorzugt atraumatisch ausgebildet, um Verletzungen und insbesondere Perforationen des Herzohrs zu verhindern. Zur besseren Verankerung können alle oder einzelne Endbereiche 9 ein weiteres Festlegungselement 9a umfassen, das bevorzugt die Form eines Widerhakens aufweist. Die verschiedenen Bereiche des Verankerungselements 6 - Hauptbereich 7, einen Nebenbereich 8 und einen Endbereich 9 - sind bevorzugt aus dem gleichen Material sowie aus einem Stück gefertigt. Desgleichen ist auch das gesamte Tentakelsystem 5 bevorzugt aus dem gleichen Material sowie aus einem Stück gefertigt.

Figur 2 zeigt das erfindungsgemäße Implantat 1 im komprimierten Zustand innerhalb der Einführeinheit 17. Die Einführeinheit 17 befindet sich in einem weiteren Katheter 16, durch den Einführeinheit 17 und Implantat 1 zum Behandlungsort vorgeschoben werden können. Die Einführeinheit 17 umfasst an ihrem distalen Ende mindestens einen röntgendichten Marker 18 zur Bestimmung der Lage der Einführeinheit 17 im Körper des Patienten. Proximal vor den Markern 18 umfasst die Einführeinheit 17 im distalen Bereich auf der Höhe des Implantats 1 mindestens eine Öffnung 19, die das innere Lumen der Einführeinheit 17 mit dem äußeren Lumen verbindet. Diese Öffnung 19 hilft vor der Intervention dabei, noch in der Einführeinheit 17 befindliche Luft heraus zu spülen.

Im komprimierten Zustand legen sich die Streben 10 des Okkluders 2 nahe an das Beabstandungssystem 4. Die Verankerungselemente 6 bilden mit dem Beabstandungssystem 4 im Wesentlichen eine nach distal weisende Linie. Die einzelnen Bereiche der Verankerungselemente 6 - Hauptbereich 7, Nebenbereich 8 und Endbereich 9 - sind mehr oder weniger gestreckt und nehmen ihre bevorzugte Winkelposition erst nach Implantation ein. Es sind neben der dargestellten Ausführungsform auch Ausführungsformen denkbar und zu bevorzugen, bei denen sich die Verankerungselemente 6 im Wesentlichen innerhalb des vom Okkluder 2 gebildeten Lumens befinden. Ebenso sind Ausführungsformen denkbar und zu bevorzugen, bei denen sich das Beabstandungssystem 4 im komprimierten Zustand innerhalb des vom Okkluder 2 gebildeten Lumens befindet.

Der proximale Bereich des Okkluders 2 umfasst ein Ablöseelement 15, mit dem der Okkluder 2 über einen Ablösemechanismus trennbar mit dem Einführelement 14 verbunden ist. Entsprechende mechanische, thermische, elektrische, elektrolytische oder sonstige Ablösemechanismen sind dem Fachmann bekannt. Bevorzugt innerhalb des Einführelements 14 verläuft das lösbar mit dem Okkluder 2 verbundene Sicherungselement 20.

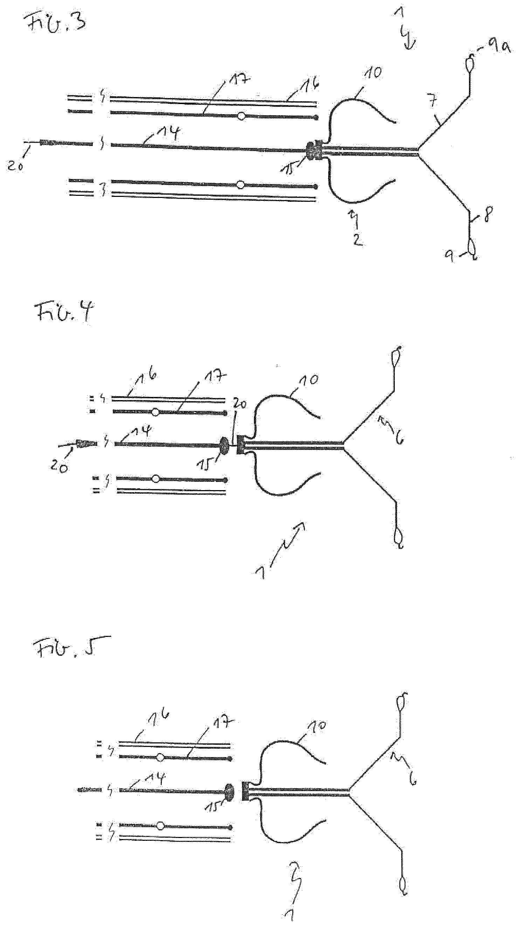

Die Figuren 3 bis 5 zeigen die wesentlichen Schritte der Freisetzung des Implantats 1 aus der Einführeinheit 17.

In Figur 3 wurde das Implantat 1 an dem Einführelement 14 aus der Einführeinheit 17 geschoben. Der Okkluder 2 nimmt einen expandierten Zustand an, die Streben 10 dehnen sich vom Beabstandungssystem 4 hinweg gegen das Eingangsgewebe des Herzohrs (nicht dargestellt). Die Verankerungselemente 6 nehmen ebenfalls ihren expandierten Zustand ein. Das Implantat 1 ist noch über das Ablöseelement 15 mit dem Einführelement 14 verbunden und kann über das Einführelement 14 zurück in die Einführeinheit 17 und/oder den weiteren Katheter 16 zurückgezogen werden, sollte eine Prüfung des korrekten Sitzes des Implantats - beispielsweise durch einen Zugtest über das Einführelement 14 - negativ ausfallen.

In Figur 4 wurde nach positiver Prüfung des Sitzes des Implantats 1 das Einführelement 14 vom Implantat 1 über das Ablöseelement 15 getrennt. Sollte sich nun nach Ablösen des Einführelements 14 zeigen, dass das Implantat nicht korrekt sitzt, kann das Implantat 1 über das Sicherungselement 20 immer noch in die Einführeinheit 17 und/oder den weiteren Katheter 16 zurückgezogen werden. Dies ist der entscheidende Vorteil der vorliegenden Erfindung hinsichtlich der Sicherheit des Implantats.

In Figur 5 ist auch das Sicherungselement 20 vom Implantat 1 gelöst. Die Implantation ist damit abgeschlossen und der weitere Katheter 16, die Einführeinheit 17 sowie das Einführelement 14 und das Sicherungselement 20 können endgültig aus dem Patienten entfernt werden.

### Bezuaszeichenliste

- 1: Implantat
- 2: Okkluder
- 3: Verankerungssystem
- 4: Beabstandungssystem/Distanzelement(e)
- 5: Tentakelsystem
- 6: Verankerungselement
- 7: Hauptbereich
- 8: Nebenbereich
- 9: Endbereich
- 9a: Festlegungselement
- 10: Streben
- 11: Befestigungselement
- 12: Festlegungsmittel des Okkluders
- 13: Festlegungsmittel des Distanzelements
- 14: Einführelement (RB)
- 15: Ablöseelement (RM)
- 16: Katheter
- 17: Einführeinheit (ODS)
- 18: Marker
- 19: Öffnungen
- 20: Sicherungselement (RL)
- α: Winkel zwischen Beabstandungssystem und Hauptbereich
- β: Winkel zwischen Haupt- und Nebenbereich

## Patentansprüche

1. Implantat (1) zum Verschließen hohler Strukturen im menschlichen oder tierischen Körper mittels endovaskulärer Intervention, umfassend
- einen Okkluder (2) mit einer aus einer Vielzahl von Streben (10) gebildeten, im expandierten Zustand im Wesentlichen korb- oder scheibenförmigen und im komprimierten Zustand im Wesentlichen rohrförmigen Struktur,
- ein Verankerungssystem (3) mit einem Beabstandungssystem (4) und einem Tentakelsystem (5) mit mindestens zwei Verankerungselementen (6), und
- ein Einführelement (14), das über ein Ablöseelement (15) trennbar mit dem proximalen Teil des Okkluders (2) verbunden ist,
**dadurch gekennzeichnet, dass** das Verankerungssystem (3) längenvariabel ist.

2. Implantat (1) nach Anspruch 1, **dadurch gekennzeichnet, dass**
- das Beabstandungssystem (4) im Wesentlichen ein rohr- und/oder stabförmig ausgestaltetes Distanzelement (4) mit einem ersten und einem zweiten Ende umfasst, wobei das Distanzelement (4) an seinem ersten Ende ein erstes Festlegungsmittel (13) umfasst, mit dem das Verankerungssystem (3) kraft- und/oder formschlüssig mit dem Okkluder (2) verbindbar ist und an seinem zweiten Ende das Tentakelsystem (5) umfasst und
- der Okkluder (2) ein zweites Festlegungsmittel (12) umfasst mit dem das Verankerungssystem (3) kraft- und/oder formschlüssig mit dem Okkluder (2) verbindbar ist.

3. Implantat (1) nach Anspruch 2, **dadurch gekennzeichnet, dass** das erste Festlegungsmittel (13) und das Distanzelement (4) aus einem Stück gefertigt sind.

4. Implantat (1) nach Anspruch 2 oder 3, **dadurch gekennzeichnet, dass** das zweite Festlegungsmittel (12) und zumindest ein Teil des Okkluders (2) aus einem Stück gefertigt sind.

5. Implantat (1) nach einem der vorherigen Ansprüche, **dadurch gekennzeichnet, dass** das erste und das zweite Festlegungsmittel (12,13) lösbar miteinander verbindbar sind.

6. Implantat (1) nach einem der vorherigen Ansprüche, **dadurch gekennzeichnet, dass** es sich bei dem ersten und zweiten Festlegungsmittel (12,13) um eine Schraubverbindung handelt, wobei das erste Festlegungsmittel (13) ein Außen- und das zweite Festlegungsmittel (12) ein dazu passendes Innengewinde umfasst.

7. Implantat (1) nach einem der vorherigen Ansprüche, **dadurch gekennzeichnet, dass** das Distanzelement (4) aus einem Rohr geschnitten oder aus Filamenten geflochten ist.

8. Implantat (1) nach einem der vorherigen Ansprüche, **dadurch gekennzeichnet, dass** das Tentakelsystem (5) zwei bis sechzehn, bevorzugt sechs bis zwölf und insbesondere acht Verankerungselemente (6) umfasst.

9. Implantat (1) nach Anspruch 8, **dadurch gekennzeichnet, dass** die Verankerungselemente (6) einen proximalen Hauptbereich (7), einen distalen Endbereich (9) und einen zwischen Haupt- und Endbereich (7,9) liegenden Nebenbereich (8) umfassen.

10. Implantat (1) nach Anspruch 9, **dadurch gekennzeichnet, dass** der Endbereich (9) atraumatisch, insbesondere in Form einer Schlaufe geformt ist, wobei der Durchmesser der Schlaufe zwischen 1 und 5 mm, insbesondere 3 mm beträgt.

11. Implantat (1) nach Anspruch 10, **dadurch gekennzeichnet, dass** der Endbereich (9) ein Festlegungsmittel (9a) umfasst, wobei das Festlegungsmittel (9a) insbesondere in Form eines Widerhakens gestaltet ist.

12. Implantat (1) nach Anspruch 10 oder 11, **dadurch gekennzeichnet, dass** der proximale Hauptbereich (7) im frei expandierten Zustand mit der durch das erste und zweite Distanzelement (4) gebildeten Achse einen nach außen gerichteten Winkel (α) zwischen 90 und 150 Grad, bevorzugt von 105 Grad bildet und der Nebenbereich (8) mit dem Hauptbereich (7) im frei expandierten Zustand einen nach außen gerichteten Winkel (β) zwischen 90 und 150 Grad, bevorzugt von 105 Grad bildet.

13. Implantat (1) nach einem der vorherigen Ansprüche, **dadurch gekennzeichnet, dass** das Tentakelsystem (5) im komprimierten Zustand im Wesentlichen innerhalb des Beabstandungssystems (4) oder unmittelbar an der Außenseite des Beabstandungssystems (4) liegt.

14. Implantat (1) nach einem der vorherigen Ansprüche, **dadurch gekennzeichnet, dass** das Implantat (1) an seinem proximalen Ende ein Befestigungselement (11) umfasst, das lösbar mit einem Sicherungselement (20) verbindbar ist, wobei das Implantat (1) mit Hilfe des Sicherungselements (20) auch nach Ablösen des Einführelements (14) in einen Katheter zurückzuziehbar ist.

15. Implantat (1) nach Anspruch 14, **dadurch gekennzeichnet, dass** das Sicherungselement (20) ein Filament umfasst und dass das Befestigungselement (11) eine Öse oder eine Öffnung umfasst, wobei das Sicherungselement (20) durch das Befestigungselement (11) führbar ist.

16. Implantat (1) nach Anspruch 14 oder 15, **dadurch gekennzeichnet, dass** das Sicherungselement (20) zumindest einmal so um oder durch das Befestigungselement (11) geführt wird, dass das Sicherungselement (20) durch Zug an einem seiner Enden, aber nicht durch Zug an beiden Enden vom Befestigungselement (11) lösbar ist.

17. Implantatsystem mit einem Implantat (1) nach einem der Ansprüche 1 bis 16, **gekennzeichnet durch** einen Satz aus zwei oder mehr Verankerungssystemen (3), die unterschiedliche Längen aufweisen.
